# EUROPÄISCHE PATENTSCHRIFT

(11) **EP 3 922 161 B1**
(45) Veröffentlichungstag und Bekanntmachung des Hinweises auf die Patenterteilung: **29.10.2025**
(21) Anmeldenummer: 21176684.5
(22) Anmeldetag: 28.05.2021
(51) Int. Cl.: A61B 1/00, A61B 1/05

(54) **ENDOSKOP MIT SCHWENKBARER BILDERFASSUNGSEINRICHTUNG**
ENDOSCOPE WITH PIVOTABLE IMAGING DEVICE
ENDOSCOPE POURVU DE DISPOSITIF PIVOTANT DE CAPTURE D'IMAGE

(30) Priorität: 09.06.2020 DE 102020115257
(43) Veröffentlichungstag der Anmeldung: 15.12.2021
(73) Patentinhaber: Karl Storz SE & Co. KG, 78532 Tuttlingen (DE)
(72) Erfinder: Forster, Jonas, 78532 Tuttlingen (DE); Heni, Andreas, 78532 Tuttlingen (DE); Kupferschmid, Markus, 78532 Tuttlingen (DE); Ulmschneider, Daniel, 78532 Tuttlingen (DE); Geafer, Lawrence, 78532 Tuttlingen (DE)

(56) Entgegenhaltungen:
- EP-A1- 3 243 426
- DE-A1- 102010 028 147
- US-A1- 2011 115 882
- US-A1- 2018 344 288
- US-A1- 2019 117 044

## Beschreibung

Die vorliegende Erfindung ist auf ein Endoskop mit schwenkbarer Bilderfassungseinrichtung bezogen.

Neben Endoskopen mit Blickrichtung parallel zur Längsachse des Schafts ("Geradeausblick") werden vor allem Endoskope, deren Blickrichtung mit der Längsachse des Schafts einen Winkel einschließen, der oft im Bereich von 30° bis 80° liegt, ("Vorausblick") verwendet. Für manche Anwendungen kommen auch Endoskope mit einem einstellbaren Winkel zwischen Blickrichtung und Längsachse des Schafts in Betracht.

In US 2007/0055103 A1 ist ein Endoskop mit variabler Blickrichtung beschrieben (Titel, Absätze [0002], [0017], [0018]). Ein optisches Abbildungssystem ("optical imaging system") 18 mit einer Abbildungsoptik ("imaging optics") 22 und einem CCD-Chip 24 ist in einem starren distalen Kopf ("rigid distal head") 16 des Endoskops 10 angeordnet (Absätze [0073], [0074]). Der distale Kopf 16 ist um eine erste Schwenkachse ("first pivot axis") 38 schwenkbar mit einem ersten Schaftabschnitt ("first shaft portion") 32 verbunden, der erste Schaftabschnitt 32 ist um eine zweite Schwenkachse 40 schwenkbar mit einem zweiten Schaftabschnitt 34 verbunden (Absätze [0079], [0082], [0083]).

In DE 10 2012 206 963 A1 ist ein Endoskop beschrieben, das einen drehbar gelagerten Halter 5 mit einer Digitalkamera 4 an oder in einer Halterung 6 aufweist, wobei die Halterung 6 radial drehbar so angeordnet ist, dass die Drehachsen der Halterung 6 und des Halters 5 senkrecht zueinander angeordnet sind (Absatz [0008]).

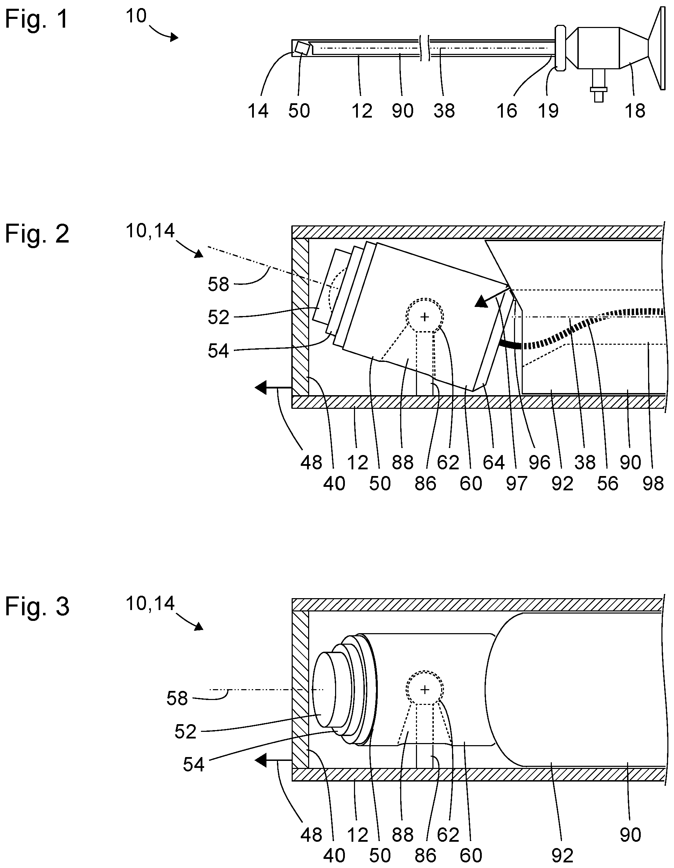

In US 2013/0182091 A1 ist ein Endoskop mit einer veränderbaren Blickrichtung beschrieben (Zusammenfassung, Absatz [0002]). Eine Abbildungseinheit ("imaging unit") 12 ist angetrieben durch Antriebsstäbe 22, 23 von zwei Antriebskraft-Übertragungsmechanismen um zwei verschiedene Achsen X, Y rotierbar (Absätze [0002], [0028], [0062], Fig. 3, 4, 6, 8, 9).

In US 2015/0359420 A1 ist ein Endoskop 1 mit einem teilweise flexiblen Schaft ("insertion section") 11 und einer Abbildungseinheit ("imaging unit") 36 am distalen Ende ("tip end portion") des Schafts 11 beschrieben (Absatz [0036], Fig. 1). Die Abbildungseinheit 6a ist schwenkbar angeordnet (Absätze [0079], [0081], Fig. 4, 5).

In EP 3 243 426 A1 ist ein Endoskop 100 mit einem länglichen Schaft ("elongated shaft") 101 und einer Bildsensoranordnung ("image sensor assembly") 202 beschrieben (Titel, Zusammenfassung, Absätze [0002], [0008], [0019], [0022], Fig. 1, 2, 5). Die Bildsensoranordnung 202 kann um eine laterale Gelenkachse ("lateral articulation axis") T1 geschwenkt werden (Absatz [0026], Fig. 2, 3, 5, 6, 7).

In DE 10 2017 103 721 A1 ist ein Stereo-Endoskop beschrieben, bei dem jeder der beiden optischen Kanäle ein Objektiv 40, 50 und eine Blickrichtungseinrichtung 20, 30 umfasst. Jede Blickrichtungseinrichtung 20, 30 ist um eine zugehörige ortsfeste Rotationsachse 48, 58 rotierbar. Eine Antriebswelle 90 ist durch Zahnräder mit den Blickrichtungseinrichtungen 20, 30 gekoppelt, um diese zu rotieren. Kurvengetriebe verschieben simultan die Blickrichtungseinrichtungen 20, 30, die Objektive 40, 50 und Bildsensoren 60, 70 parallel zu den Rotationsachsen.

In WO 2018/065241 A1 ist ein Stereo-Videoendoskop 2 mit einem optischen System 20 beschrieben (Titel, Zusammenfassung, Seite 13, Zeile 26, bis Seite 14, Zeile 7, Seite 14, Zeilen 27 bis 29, Fig. 1, 2). Durch Drehen eines Handgriffs 4 kann die Blickrichtung um die Längsachse des Endoskopschafts 6 rotiert werden (Seite 14, Zeilen 18 bis 21). Zur Beibehaltung der Horizontallage des angezeigten Bilds wird bei einer Drehung des Handgriffs 4 ein Drehrad 14 festgehalten, dies bewirkt, dass die Bildsensoren 52L, 52R im Inneren des Endoskopschafts 6 die Drehbewegung nicht mitvollziehen (Seite 14, Zeilen 21 bis 25, Seite 16, Zeilen 2 bis 13, Fig. 2).

In US 2019/0274526 A1 ist ein Stereoendoskop 1 mit einer Bilderfassungsvorrichtung ("image pick-up apparatus") 30 in einem distalen Endabschnitt ("distal end portion") 11 distal eines biegbaren Abschnitts ("bending portion") des Schafts ("long insertion portion") 2 beschrieben (Absätze [0021], [0023], [0025], Fig. 1, 2)

In US 2019/0117044 A1 ist ein Kathetersystem mit einem Schaft und einer Kameraanordnung mit einstellbarer Blickrichtung an dem distalen Ende des Schafts beschrieben. Ein Kugelgelenk, eine Schraubfeder und ein Drehmomentstab ermöglichen ein Einstellen der Blickrichtung einer Kamera wobei die Schraubfeder den Drehmomentstab mit dem Kugelgelenk verbindet.

Bei einem Rotieren einer von der Längsachse des distalen Endes des Schafts abweichenden Blickrichtung auf einen Kegelmantel um die Längsachse des distalen Endes des Schafts wird auch das von dem Endoskop erzeugte Bild relativ zu dem Endoskop rotiert. Bei einem analogen, das heißt rein optischen Endoskop, mit dessen Okular eine Kamera verbunden ist, kann dies durch eine gegenläufige Rotation der Kamera oder anders ausgedrückt durch ein Festhalten der Kamera während des Rotierens des Endoskops vermieden werden. Bei einem monokularen Videoendoskop kann das vom mitrotierenden Bildsensor erfasste Bild digital zurückrotiert werden. Bei einem Stereo-Videoendoskop rotiert jedoch mit dem Endoskop auch die Stereobasis. Auch dies kann digital kompensiert werden, indem aus dem Stereobild Tiefeninformation berechnet, also räumliche Information gewonnen und aus dieser ein Stereobild mit der gewünschten Basis synthetisiert wird. Allerdings sind der Rechenaufwand hoch und in vielen Situationen Artefakte und Bildstörungen hinzunehmen.

Eine Aufgabe der vorliegenden Erfindung besteht darin, ein verbessertes, insbesondere mechanisch robustes und miniaturisierbares Endoskop oder Exoskop mit bewegbarer Blickrichtung zu schaffen.

Diese Aufgabe wird gelöst durch den Gegenstand des Anspruchs 1.

Weitere Ausführungsformen sind in den abhängigen Ansprüchen definiert.

Ausführungsformen der vorliegenden Erfindung beruhen auf der Idee, eine schwenkbare Bilderfassungseinrichtung an einem distalen Ende eines Endoskops oder eines Exoskops mittels einer rotierbaren Antriebseinrichtung zu schwenken. Die rotierbare Antriebseinrichtung weist insbesondere ein zu ihrer Rotationsachse nicht rotationssymmetrisches distales Ende auf, das unmittelbar oder mittelbar an der Bilderfassungseinrichtung angreift und die Bilderfassungseinrichtung abhängig von ihrer eigenen rotatorischen Position, insbesondere dieser entsprechend, schwenkt.

Ein Endoskop oder Exoskop umfasst eine Bilderfassungseinrichtung mit einem Objektiv zum Erzeugen eines reellen Bilds und einen Bildsensor zum Erfassen des reellen Bilds und zum Erzeugen eines Bildsignals, das das erfasste reelle Bild repräsentiert, eine Schwenklagereinrichtung, die ein Bewegen der optischen Achse des Objektivs der Bilderfassungseinrichtung auf einem Kegelmantel ermöglicht, und eine um eine Rotationsachse rotierbare Antriebseinrichtung, wobei die rotierbare Antriebseinrichtung derart mit der Bilderfassungseinrichtung mechanisch gekoppelt ist, dass eine Rotation der rotierbaren Antriebseinrichtung mit einem Bewegen der optischen Achse des Objektivs der Bilderfassungseinrichtung auf einem Kegelmantel einhergeht.

Ein Exoskop ist eine zum extrakorporalen Gebrauch vorgesehene und ausgebildete Vorrichtung zur visuellen Inspektion bzw. Betrachtung von Objekten in der Medizin, insbesondere von Objekten an oder nahe äußeren Oberflächen eines menschlichen oder tierischen Körpers. Im Unterschied zu einem Endoskop ist ein Exoskop nicht dazu ausgebildet, um durch eine kleine natürliche oder künstliche Öffnung in einen natürlichen oder künstlichen Hohlraum eingeführt zu werden. Ein Exoskop ist vielmehr für eine Betrachtung eines Objekts ausgebildet, das zumindest während der Betrachtung, insbesondere während einer Operation, von außen sichtbar ist. Entsprechend befindet sich das Exoskop während seines bestimmungsgemäßen Einsatzes vollständig außerhalb des menschlichen oder tierischen Körpers und weist im Unterschied zum Endoskop nicht unbedingt einen langen dünnen Schaft auf.

Ein Exoskop ist in der Regel für eine Gegenstandsweite im Bereich von einigen oder wenigen Zentimetern oder wenigen Dezimetern ausgebildet oder optimiert. Ein Exoskop kann eine starke Vergrößerung aufweisen, die eine Auflösung ermöglicht, die mit bloßem Auge nicht erreichbar ist, und damit Eigenschaften einer Lupe bzw. Stereolupe oder eines Mikroskops bzw. Stereomikroskops aufweisen. Vom Mikroskop bzw. Stereomikroskop kann sich das Exoskop durch eine größere Gegenstandsweite unterscheiden.

Ein Endoskop, wie es hier beschrieben ist, umfasst insbesondere einen Schaft mit einem proximalen Ende und einem distalen Ende zum Einführen in einen Hohlraum, wobei die Bilderfassungseinrichtung an dem distalen Ende des Schafts angeordnet ist. Das distale Ende des Schafts kann durch ein optisch transparentes Fensterbauteil fluiddicht, insbesondere hermetisch verschlossen sein. Wenn das Endoskop ein Fensterbauteil aufweist, ist die Bilderfassungseinrichtung insbesondere unmittelbar proximal des Fensterbauteils angeordnet.

Die Schwenklagereinrichtung ist insbesondere ausgebildet, um ein Bewegen der optischen Achse innerhalb eines Raumwinkelbereichs zu ermöglichen. Innerhalb des Bereichs in der aufgrund der Schwenklagereinrichtung an sich erreichbaren Orientierungen der Bilderfassungseinrichtung steuert die Antriebseinrichtung insbesondere eine Bewegung der optischen Achse des Objektivs der Bilderfassungseinrichtung auf einem vollständigen Kegelmantel oder auf einem Teilbereich eines Kegelmantels mit einem vorbestimmten Öffnungswinkel. Der Kegelmantel ist insbesondere rotationssymmetrisch zu einer Längsachse und/oder Symmetrieachse des distalen Endes des Schafts. Alternativ kann eine Symmetrieachse des Kegelmantels gegenüber der Längs- und/oder Symmetrieachse des distalen Endes des Schafts gekippt sein.

Die Rotationsachse der rotierbaren Antriebseinrichtung ist insbesondere gleich der Längsachse des Schafts oder des distalen Endbereichs des Schafts. Die rotierbare Antriebseinrichtung erstreckt sich insbesondere entlang des gesamten Schafts oder eines großen Teils des Schafts von einer Handhabungseinrichtung am proximalen Ende des Schafts bis zu der Bilderfassungseinrichtung am distalen Ende des Schafts. Das proximale Ende der rotierbaren Antriebseinrichtung kann mit einem manuell bedienbaren Bedienelement an einer Handhabungseinrichtung in einem proximalen Endbereich des Endoskops unmittelbar mechanisch verbunden oder mittelbar mechanisch oder magnetisch gekoppelt sein.

Beispielsweise weist das proximale Ende der rotierbaren Antriebseinrichtung einen oder mehrere Magneten auf, die zusammen mit der gesamten rotierbaren Antriebseinrichtung innerhalb einer hermetisch dichten Hülle des Endoskops oder Exoskops angeordnet sind. Ein Rad oder eine andere manuell bewegbare Steuerungseinrichtung weist einen oder mehrere Magneten auf, der bzw. die mit dem oder den Magneten am proximalen Ende der rotierbaren Antriebseinrichtung derart wechselwirken, dass eine - insbesondere unmittelbar manuell erzeugte - Bewegung der Steuereinrichtung eine Rotation der Antriebseinrichtung bewirkt.

Das distale Ende der rotierbaren Antriebseinrichtung ist insbesondere nicht rotationssymmetrisch zu der Rotationsachse der rotierbaren Antriebseinrichtung. Eine unmittelbare oder mittelbare mechanische Wechselwirkung zwischen dem distalen Ende der rotierbaren Antriebseinrichtung und der Bilderfassungseinrichtung erzwingt bei einer Rotation der Antriebseinrichtung eine entsprechende Rotation der Blickrichtung der Bilderfassungseinrichtung.

Bei einem Endoskop oder Exoskop, wie es hier beschrieben ist, weist die rotierbare Antriebseinrichtung insbesondere kein Zahnrad, kein Kurvengetriebe und keine Schnecke auf.

Bei einem Endoskop oder Exoskop, wie es hier beschrieben ist, ist insbesondere ein distales Ende der Antriebseinrichtung zumindest entweder proximal der Schwenklagereinrichtung oder proximal der Bilderfassungseinrichtung angeordnet.

Das distale Ende der Antriebseinrichtung ist insbesondere dann proximal der Bilderfassungseinrichtung angeordnet, wenn es in Längsrichtung des Schafts nicht oder nur geringfügig - also mit weniger als der Hälfte oder weniger als einem Viertel der Längserstreckung der Bilderfassungseinrichtung - überlappt.

Bei einem Endoskop oder Exoskop, wie es hier beschrieben ist, ermöglicht die Schwenklagereinrichtung insbesondere ein Schwenken um eine erste Schwenkachse und ein Schwenken um eine zweite Schwenkachse, die von der ersten Schwenkachse verschieden ist.

Bei einem Endoskop oder Exoskop, wie es hier beschrieben ist, ermöglicht die Schwenklagereinrichtung insbesondere ein Schwenken um eine erste Schwenkachse orthogonal oder im Wesentlichen orthogonal zu einer Symmetrieachse des Kegelmantels und ein Schwenken um eine zweite Schwenkachse orthogonal oder im Wesentlichen orthogonal zu der ersten Schwenkachse und orthogonal oder im Wesentlichen orthogonal zu der Symmetrieachse des Kegelmantels.

Die erste Schwenkachse ist im Wesentlichen orthogonal zu der Symmetrieachse des Kegelmantels, wenn ein Winkel zwischen der ersten Schwenkachse und der Symmetrieachse des Kegelmantels nicht kleiner als 70° oder nicht kleiner als 80° oder nicht kleiner als 85° ist. Die zweite Schwenkachse ist zu der ersten Schwenkachse insbesondere orthogonal oder im Wesentlichen orthogonal, wenn ein Winkel zwischen der zweiten Schwenkachse und der ersten Schwenkachse nicht kleiner als 70° oder nicht kleiner als 80° oder nicht kleiner als 85° ist. Der Winkel zwischen der ersten Schwenkachse und der zweiten Schwenkachse ist insbesondere konstant. Bei einem Schwenken der Bilderfassungseinrichtung um die erste Schwenkachse wird die zweite Schwenkachse insbesondere ebenso geschwenkt.

Ein Endoskop oder Exoskop, wie es hier beschrieben ist, umfasst insbesondere ferner eine Führungseinrichtung, die eine vorbestimmte Reihe von Pixeln des Bildsensors in jeder Position der Bilderfassungseinrichtung parallel zu einer vorbestimmten Ebene hält.

Die Führungseinrichtung kann teilweise oder vollständig mit der Schwenklagereinrichtung integriert oder identisch sein. Die vorbestimmte Ebene ist insbesondere orthogonal zu einer durch die Schwenklagereinrichtung definierten ersten, feststehenden Schwenkachse. Die vorbestimmte Reihe von Pixeln ist insbesondere eine Zeile oder eine Spalte von Pixeln parallel zu einem geraden Randabschnitt eines rechteckigen Bildsensors. Die Reihe von Pixeln wird auf einem Bildschirm insbesondere entlang einer horizontalen oder vertikalen Geraden dargestellt.

Die Führungseinrichtung unterbindet eine Rotation der Bilderfassungseinrichtung um die Symmetrieachse des Kegelmantels, im Fall eines Endoskops insbesondere um die Längsachse des distalen Endes des Schafts. Damit gewährleistet die Führungseinrichtung, dass auch bei einem Schwenken der Bilderfassungseinrichtung und damit der Blickrichtung der Horizont unverändert bleibt und eine Bildaufrichtung nicht erforderlich ist.

Bei einem Endoskop oder Exoskop, wie es hier beschrieben ist, umfasst die Schwenklagereinrichtung insbesondere eine kardanische Aufhängung.

Die kardanische Aufhängung definiert insbesondere zwei orthogonale Schwenkachsen und wirkt gleichzeitig als Führungseinrichtung, die eine vorbestimmte Reihe von Pixeln des Bildsensors jederzeit parallel zu einer vorbestimmten Ebene hält.

Bei einem Endoskop oder Exoskop, wie es hier beschrieben ist, umfasst die Schwenklagereinrichtung insbesondere ein Kugelgelenk, ein stabförmiges Bauteil, an dessen Ende das Kugelgelenk angeordnet ist, und einen Schlitz, in dem das stabförmige Bauteil spiel- und reibungsarm schwenkbar geführt ist.

Im Fall eines Endoskops ist das Kugelgelenk insbesondere näherungsweise in der Mitte des Querschnitts des Schafts angeordnet. Die Führung des stabförmigen Bauteils in dem Schlitz bewirkt, dass eine Reihe von Pixeln des Bildsensors in einer Ebene orthogonal zu dem stabförmigen Bauteil beim Schwenken der Bilderfassungseinrichtung und der Blickrichtung stets parallel zu der Ebene bleibt.

Ein Endoskop oder Exoskop, wie es hier beschrieben ist, umfasst insbesondere ferner eine erste Gleitfläche, die mit der Bilderfassungseinrichtung mechanisch starr verbunden ist, und eine zweite Gleitfläche an einem distalen Ende der rotierbaren Antriebseinrichtung, die an der ersten Gleitfläche anliegt.

Die erste Gleitfläche ist beispielsweise rotationssymmetrisch zur Blickrichtung der Bilderfassungseinrichtung. Die zweite Gleitfläche ist nicht rotationssymmetrisch zur Rotationsachse der Antriebseinrichtung. Die erste Gleitfläche ist insbesondere ein Teilbereich der äußeren Oberfläche eines Trägers oder Gehäuses der Bilderfassungseinrichtung. Die zweite Gleitfläche ist mit der rotierbaren Antriebseinrichtung insbesondere starr verbunden und beispielsweise durch einen Oberflächenbereich an dem distalen Ende der rotierbaren Antriebseinrichtung gebildet.

Bei einem Endoskop oder Exoskop, wie es hier beschrieben ist, ist die erste Gleitfläche insbesondere kreisbogenförmig oder kreisringförmig.

Wenn die Blickrichtung der Bilderfassungseinrichtung entlang eines vollständigen Kegelmantels um 360° schwenkbar ist, ist die erste Gleitfläche insbesondere kreisringförmig. Wenn die Blickrichtung der Bilderfassungseinrichtung nur über einen Teil eines Kegelmantels bewegbar ist, kann die erste Gleitfläche C-förmig ausgebildet sein.

Bei einem Endoskop oder Exoskop, wie es hier beschrieben ist, ist die erste Gleitfläche insbesondere rotationssymmetrisch zur Blickrichtung der Bilderfassungseinrichtung.

Bei einem Endoskop oder Exoskop, wie es hier beschrieben ist, umfasst die zweite Gleitfläche insbesondere zwei voneinander beabstandete Teilflächen.

Die zwei Teilflächen der zweiten Gleitfläche sind insofern voneinander beabstandet, als zwischen den zwei Teilflächen der zweiten Gleitfläche keine Berührung zwischen der rotierbaren Antriebseinrichtung oder einem mit der rotierbaren Antriebseinrichtung mechanisch gekoppelten Körper, an dem die zweite Gleitfläche vorgesehen ist, und der ersten Gleitfläche stattfindet.

Die Aufteilung der zweiten Gleitfläche in zwei voneinander beabstandete Teilflächen kann eine besonders präzise mechanische Kopplung der rotierbaren Antriebseinrichtung mit der Bilderfassungseinrichtung und damit eine besonders präzise Steuerung der Blickrichtung der Bilderfassungseinrichtung ermöglichen.

Bei einem Endoskop oder Exoskop, wie es hier beschrieben ist, sind insbesondere die erste Gleitfläche nach proximal und die zweite Gleitfläche nach distal orientiert.

Insbesondere ist eine Flächennormale der ersten Gleitfläche parallel zu einer Rotationsachse der rotierbaren Antriebseinrichtung oder schließt mit dieser einen Winkel ein, der nicht größer als 30° oder nicht größer als 45° ist. Die Flächennormale der zweiten Gleitfläche ist insbesondere antiparallel zu der Flächennormale der ersten Gleitfläche. Bei einer Orientierung nach distal bzw. proximal ist die zweite Gleitfläche insbesondere nahe dem äußeren Umfang der rotierbaren Antriebseinrichtung und im Fall eines Endoskops nahe dem äußeren Umfang des Lumens des Schafts des Endoskops angeordnet.

Bei einem Endoskop oder Exoskop, wie es hier beschrieben ist, ist eine Flächennormale der zweiten Gleitfläche insbesondere zu einer Rotationsachse der rotierbaren Antriebseinrichtung hin orientiert.

Die Flächennormale der ersten Gleitfläche ist entgegengesetzt parallel zu der Flächennormale der zweiten Gleitfläche. Die Flächennormale der zweiten Gleitfläche ist insbesondere radial oder im Wesentlichen radial zu der Rotationsachse der rotierbaren Antriebseinrichtung angeordnet. Dies bedeutet, dass die Flächennormale der zweiten Gleitfläche am Ort jeder Berührung mit der ersten Gleitfläche in einer Ebene mit der Rotationsachse der rotierbaren Antriebseinrichtung liegt und mit dieser einen Winkel einschließt, der nicht größer als 45° oder nicht größer als 30° oder nicht größer als 20° oder nicht größer als 10° ist.

Bei einem Endoskop oder Exoskop, wie es hier beschrieben ist, sind insbesondere zwei gleiche oder ähnliche Bilderfassungseinrichtungen nebeneinander angeordnet und miteinander mechanisch starr verbunden.

Das Endoskop oder Exoskop kann ferner eine dritte und optional weitere Bilderfassungseinrichtungen aufweisen, die miteinander und mit der ersten und der zweiten Bilderfasungseinrichtung mechanisch starr verbunden sein können. Die zwei oder mehr miteinander mechanisch starr verbundenen Bilderfassungseinrichtungen sind insbesondere auf einem gemeinsamen Träger oder in einem gemeinsamen Gehäuse angeordnet. Die Blickrichtungen der zwei oder mehr miteinander mechanisch starr verbundenen Bilderfassungseinrichtungen sind insbesondere parallel oder leicht konvergent. Die zwei oder mehr miteinander mechanisch starr verbundenen Bilderfassungseinrichtungen ermöglichen ein Erfassen eines Stereobilds aus einem für die Wahrnehmung mit dem linken Auge vorgesehenen Bild und einem für die Wahrnehmung mit dem rechten Auge vorgesehenen Bild.

Bei einem Endoskop oder Exoskop, wie es hier beschrieben ist, umfasst die Schwenklagereinrichtung insbesondere ein Kugelgelenk, das zwischen den Bilderfassungseinrichtungen angeordnet ist.

Ein Endoskop, wie es hier beschrieben ist, umfasst insbesondere ferner einen Schaft mit einem proximalen Ende und einem distalen Ende zum Einführen in einen Hohlraum, eine Signalleitung zumindest entweder zum Übertragen eines Steuersignals von dem proximalen Ende des Schafts zu der Bilderfassungseinrichtung oder zum Übertragen eines Bildsignals von der Bilderfassungseinrichtung zu dem proximalen Ende des Schafts, wobei die rotierbare Antriebseinrichtung als Rohr ausgebildet ist, wobei die Signalleitung innerhalb eines Lumens der rotierbaren Antriebseinrichtung angeordnet ist.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine schematische Darstellung des distalen Endes des Endoskops aus Figur 1;
- Figur 3: eine weitere schematische Darstellung des distalen Endes des Endoskops aus den Figuren 1 und 2;

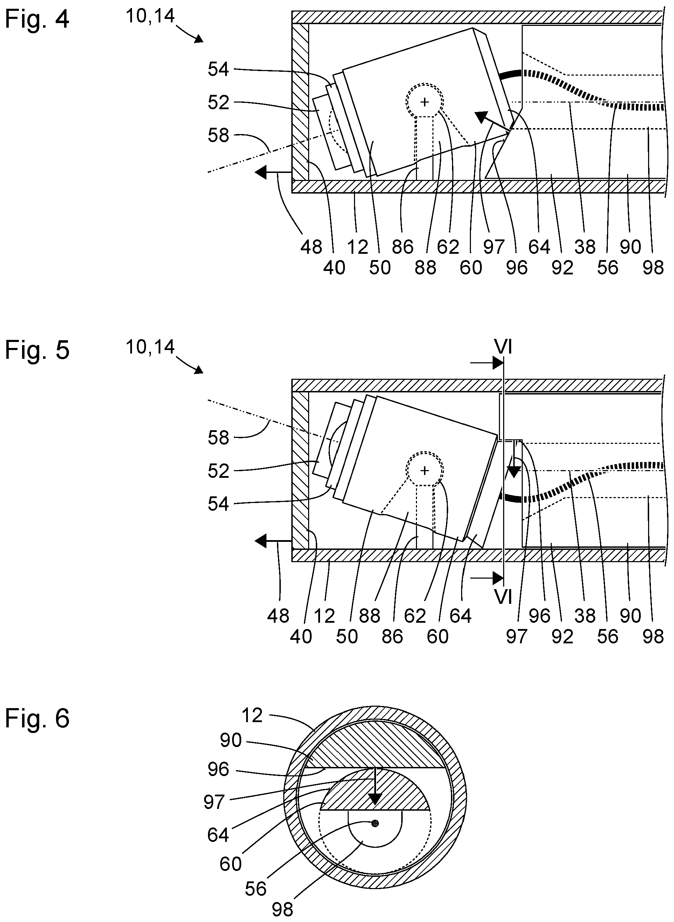

- Figur 4: eine weitere schematische Darstellung des distalen Endes des Endoskops aus den Figuren 1 bis 3;
- Figur 5: eine schematische Darstellung des distalen Endes eines weiteren Endoskops;
- Figur 6: eine weitere schematische Darstellung des distalen Endes des Endoskops aus Figur 5;

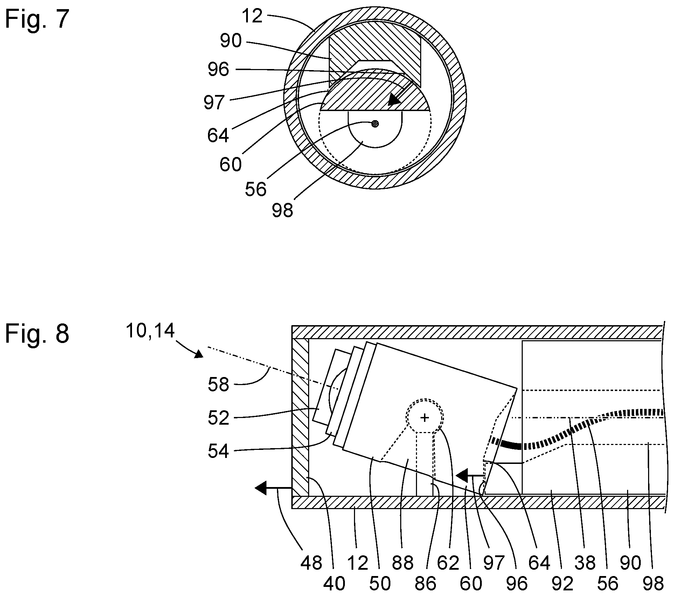

- Figur 7: eine schematische Darstellung des distalen Endes eines weiteren Endoskops;
- Figur 8: eine schematische Darstellung des distalen Endes eines weiteren Endoskops.

### Beschreibung der Ausführungsformen

Figur 1 zeigt eine schematische Darstellung eines Endoskops 10 mit einem Schaft 12. Der Schaft 12 weist ein distales Ende 14 zum Einführen in einen Hohlraum und ein proximales Ende 16 auf. Bei dem dargestellten Beispiel ist der Schaft 12 gerade und starr ausgebildet. Alternativ kann der Schaft 12 abschnittsweise oder vollständig gekrümmt oder krümmbar ausgebildet sein.

Ein proximaler Bereich 18 des Endoskops 10 ist mit dem proximalen Ende 16 des Schafts 12 verbunden und als Handhabungseinrichtung ausgebildet. Der proximale Bereich 18 des Endoskops 10 weist beispielsweise eine Steckkupplung zum Anschluss eines Lichtleitkabels zum Zuführen von Beleuchtungslicht und ein Bedienelement 19 auf. Das Bedienelement 19 ist bei dem dargestellten Beispiel als Drehrad im Übergangsbereich zwischen dem proximalen Bereich 18 des Endoskops 10 und dem proximalen Ende 16 des Schafts 12 ausgebildet. Das Drehrad 19 ist um die Längsachse 38 des Schafts 12 rotierbar.

In Figur 1 sind innerhalb der Kontur des Schafts 12 Bauteile angedeutet, die von dem Schaft 12 umschlossen, insbesondere hermetisch dicht umschlossen sind. Dies sind insbesondere eine Bilderfassungseinrichtung 50 am distalen Ende 14 des Schafts 12 und eine Antriebseinrichtung 90, die sich von dem proximalen Bereich 18 des Endoskops 10 bis zu der Bilderfassungseinrichtung 50 erstreckt.

Figur 2 zeigt eine gegenüber Figur 1 vergrößerte schematische Darstellung des distalen Endes 14 des Schafts 12 des Endoskops 10 aus Figur 1. Der Schaft 12 ist in Figur 2 im Schnitt entlang einer Ebene, die die Längsachse 38 des Schafts 12 enthält, dargestellt. Die Bilderfassungseinrichtung 50 und die Antriebseinrichtung 90 sind jeweils in seitlicher Draufsicht dargestellt. Einzelne Merkmale der Bilderfassungseinrichtung 50 und der Antriebseinrichtung 90, die von außen nicht sichtbar, aber für die im Folgenden beschriebene Funktionalität von Bedeutung sind, sind in gestrichelten Linien angedeutet.

Das distale Ende 14 des Schafts 12 weist im Wesentlichen die Gestalt eines Rohres mit kreisförmigem Querschnitt auf, das durch ein Fensterbauteil 40 nach distal optisch transparent, aber fluiddicht und insbesondere hermetisch dicht verschlossen ist. Bei dem dargestellten Beispiel ist das Fensterbauteil 40 orthogonal zur Längsachse 38 des Schafts 12 orientiert, die Flächennormale 48 des Fensterbauteils 40 ist parallel zu der Längsachse 38 des Schafts 12.

Die Bilderfassungseinrichtung 50 ist am distalen Ende 14 des Schafts 12 und unmittelbar proximal des Fensterbauteils 40 angeordnet. Die Bilderfassungseinrichtung 50 weist ein Objektiv 52 zum Erzeugen eines reellen Bilds und einen Bildsensor 54 zum Erfassen des von dem Objektiv 52 erzeugten reellen Bilds und zum Erzeugen eines Bildsignals, das das erfasste Bild analog oder digital repräsentiert, auf. Eine Signalleitung 56 verbindet den Bildsensor 54 mit dem proximalen Bereich 18 des Endoskops 10 (vgl. Figur 1). Die Signalleitung 56 überträgt Leistung und Steuersignale zu dem Bildsensor 54 und das Bildsignal des Bildsensors 54 zu dem proximalen Bereich 18 des Endoskops 10. Das Objektiv 52 weist eine oder mehrere gekrümmte lichtbrechende Flächen auf, von denen eine in Figur 2 durch eine gestrichelte Linie angedeutet ist, und eine optische Achse 58. Die optische Achse 58 repräsentiert gleichzeitig die Blickrichtung der Bilderfassungseinrichtung 50.

Die Bilderfassungseinrichtung 50 umfasst ein Gehäuse 60, das über eine Schwenklagereinrichtung 62 mit dem Schaft 12 verbunden ist. Die Schwenklagereinrichtung 62 ist als Kugelgelenk im Inneren des Gehäuses 60 ausgebildet und deshalb nur durch gestrichelte Linien angedeutet. Die Schwenklagereinrichtung 62 ermöglicht ein Schwenken der Bilderfassungseinrichtung 50 und der Blickrichtung 58 um zwei Schwenkachsen orthogonal zu der Längsachse 38 des Schafts 12. Die Schwenkbewegungen der Bilderfassungseinrichtung 50 sind durch die Geometrie des Lumens des Schafts 12 und die Geometrie der Bilderfassungseinrichtung 50, insbesondere ihres Gehäuses 60 auf einen kegelförmigen oder näherungsweise einen kegelförmigen Raumwinkelbereich beschränkt.

Die Bilderfassungseinrichtung 50 weist eine Gleitfläche 64 an dem Gehäuse 60 auf. Bei dem dargestellten Beispiel weist die Gleitfläche 64 die Gestalt eines kreisringförmigen Ausschnitts aus der Mantelfläche eines Kreiskegels auf.

Das die Schwenklagereinrichtung bildende Kugelgelenk 62 ist über ein stabförmiges Bauteil 86 mechanisch starr mit dem Schaft 12 des Endoskops 10 verbunden. Das stabförmige Bauteil 86 ist in einem Schlitz 88 in dem Gehäuse 60 der Bilderfassungseinrichtung 50 angeordnet. Die Abmessungen des stabförmigen Bauteils 86, insbesondere der Durchmesser seines Querschnitts, und die Abmessungen des Schlitzes 88 in dem Gehäuse 60 sind so aufeinander abgestimmt, dass das stabförmige Bauteil 86 spiel- und reibungsarm in dem Schlitz 88 geführt ist. Das stabförmige Bauteil 86 und der Schlitz 88 bilden somit eine Führungseinrichtung, die die durch das Kugelgelenk 62 an sich geschaffenen Freiheitsgrade von 3 auf 2 reduziert.

Aufgrund der Führung des stabförmigen Bauteils 86 in dem Schlitz 88 in dem Gehäuse 60 kann die Bilderfassungseinrichtung 50 nicht um ihre Blickrichtung rotiert werden. Deshalb bleiben bestimmte Reihen - insbesondere Zeilen - von Pixeln des Bildsensors 54, nämlich alle Reihen von Pixeln, die orthogonal zu der Ebene des Schlitzes 88 sind, jederzeit parallel zu einer Ebene, die orthogonal zu der Längsachse des stabförmigen Bauteils 86 ist. Ein Schwenken der Blickrichtung 58 der Bilderfassungseinrichtung 50 geht deshalb nicht mit einer Rotation des von dem Bildsensor 54 erfassten Bilds einher.

Die Antriebseinrichtung 90 ist um die Längsachse 38 des Schafts 12 rotierbar. Am distalen Ende 92 der Antriebseinrichtung 90 ist eine Gleitfläche 96 vorgesehen, die an der Gleitfläche 64 der Bilderfassungseinrichtung 50 anliegt. Die Flächennormale 97 der Gleitfläche 96 ist bei dem dargestellten Beispiel näherungsweise nach distal orientiert. Abweichend von der schematischen Darstellung in Figur 2 kann der Abstand zwischen den durch die Schwenklagereinrichtung 62 definierten Schwenkachsen und einer orthogonalen Geraden durch den Berührungspunkt der Gleitflächen 64, 96 deutlich größer sein.

Die Antriebseinrichtung 90 ist im Wesentlichen rohrförmig ausgebildet mit einem Lumen 98, in dem die Signalleitung 56 angeordnet ist. Bei dem dargestellten Beispiel ist das Lumen 98 in der Antriebseinrichtung 90 am distalen Ende 92 der Antriebseinrichtung 90 erweitert, um Bauraum für die Signalleitung 56 zu schaffen.

Figur 3 zeigt eine weitere schematische Darstellung des distalen Endes 14 des Endoskops 10 aus den Figuren 1 und 2. Die Art der Darstellung, insbesondere die Lage der Schnitt- und Zeichenebene, entspricht derjenigen der Figur 2.

In Figur 3 ist eine Situation gezeigt, in der die Antriebseinrichtung 90 gegenüber der in Figur 2 gezeigten Konfiguration um 90° rotiert ist, und zwar von distal aus gesehen im Uhrzeigersinn. Entsprechend ist die Blickrichtung 58 der Bilderfassungseinrichtung 50 anders orientiert. Während bei der in Figur 2 gezeigten Konfiguration die Blickrichtung 58 parallel zur Zeichenebene der Figur 2 ist, ist bei der in Figur 3 gezeigten Konfiguration aus der Zeichenebene heraus zum Betrachter hin geschwenkt.

Figur 4 zeigt eine weitere schematische Darstellung des distalen Endes 14 des Schafts 12 des Endoskops 10 aus den Figuren 1 bis 3. Die Art der Darstellung, insbesondere die Lage der Schnitt- und Zeichenebenen, entspricht derjenigen der Figuren 2 und 3.

In Figur 4 ist eine Konfiguration gezeigt, in der die Antriebseinrichtung 90 gegenüber der in Figur 3 gezeigten Konfiguration um weitere 90° weitergedreht ist. Die Positionen der Antriebseinrichtung 90 in den in den Figuren 2 und 4 gezeigten Konfigurationen unterscheiden sich also um 180°. Entsprechend der anderen rotatorischen Position der Antriebseinrichtung 90 ist auch die Blickrichtung 58 der Bilderfassungseinrichtung 50 auf einem Kegelmantel weitergeschwenkt und liegt wieder parallel zur Zeichenebene der Figur 4.

Durch Rotation der Antriebseinrichtung 90 - insbesondere durch manuelle Rotation des Drehrads 19 (vgl. Figur 1) - um die Längsachse 38 des Schafts 12 des Endoskops 10 kann die Blickrichtung 58 der Bilderfassungseinrichtung 50 geschwenkt werden und beliebige Positionen auf einem Kegelmantel einnehmen.

Figur 5 zeigt eine schematische Darstellung eines distalen Endes 14 eines Schafts 12 eines weiteren Endoskops 10, das in einigen Merkmalen, Eigenschaften und Funktionen dem anhand der Figuren 1 bis 4 dargestellten Endoskop ähnelt. Nachfolgend sind insbesondere Merkmale, Eigenschaften und Funktionen des in Figur 5 gezeigten Endoskops 10 beschrieben, in denen dieses sich von dem anhand der Figuren 1 bis 4 dargestellten Endoskop unterscheidet. Die Art der Darstellung in Figur 5 entspricht derjenigen der Figuren 2 bis 4.

Das in Figur 5 gezeigte Endoskop 10 unterscheidet sich von dem anhand der Figuren 1 bis 4 dargestellten Endoskop insbesondere durch eine andere Orientierung der Gleitflächen 64, 96 an Bilderfassungseinrichtung 50 und Antriebseinrichtung 90. Die Flächennormale 97 der Gleitfläche 96 am distalen Ende 92 der Antriebseinrichtung 90 ist radial zur Längsachse 38 des Schafts 12, die auch Rotationsachse der rotierbaren Antriebseinrichtung 90 ist, orientiert. Die Gleitfläche 64 am Gehäuse 60 der Bilderfassungseinrichtung 50 ist ein kreisringförmiger Ausschnitt aus einer Mantelfläche eines spitzen Kreiskegels.

Figur 6 zeigt eine weitere schematische Darstellung des distalen Endes 14 des Schafts 12 des Endoskops 10 aus Figur 5. In Figur 6 ist ein Schnitt entlang der in Figur 5 angedeuteten Ebene VI-VI orthogonal zu der Längsachse 38 des Schafts 12 gezeigt. Der Schaft 12 weist die Gestalt eines Rohrs mit kreisförmigem Querschnitt auf. Die Antriebseinrichtung 50 ist im Wesentlichen kreiszylindrisch und im Lumen des Schafts 12 spiel- und reibungsarm geführt.

Die Gleitfläche 96 ist an einer wie bereits in Figur 5 erkennbar nach distal überstehenden Nase der Antriebseinrichtung 90 angeordnet. Das Gehäuse 60 der Bilderfassungseinrichtung 50 und die Gleitfläche 64 an dem Gehäuse 60 sind durch die Schnittebene VI-VI nur angeschnitten. Der vor der Schnittebene VI-VI liegende und deshalb an sich in der Darstellung in Figur 6 nicht sichtbare Abschnitt des Randes der Gleitfläche 64 ist in Figur 6 durch eine gestrichelte Linie angedeutet. Der distale Rand des als Kabelkanal für die Signalleitung 56 fungierenden Lumens 98 in der Antriebseinrichtung 90 ist hinter dem Gehäuse 60 der Bilderfassungseinrichtung 50 teilweise sichtbar.

Figur 7 zeigt eine schematische Darstellung eines Schnitts durch einen Schaft 12 eines Endoskops, das in einigen Merkmalen, Eigenschaften und Funktionen dem anhand der Figuren 1 bis 4 dargestellten Endoskop und vor allem dem anhand der Figuren 5 und 6 dargestellten Endoskop ähnelt. Nachfolgend sind insbesondere Merkmale, Eigenschaften und Funktionen beschrieben, in denen das in Figur 7 gezeigte Endoskop sich von dem anhand der Figuren 5 und 6 dargestellten Endoskop unterscheidet. Die Art der Darstellung in Figur 7, insbesondere die Orientierung und Lage der dargestellten Schnittebene, entspricht derjenigen der Figur 6.

Das in Figur 7 dargestellte Endoskop unterscheidet sich von dem anhand der Figuren 5 und 6 dargestellten Endoskop insbesondere dadurch, dass die Gleitfläche 96 am distalen Ende der Antriebseinrichtung 90 aus zwei voneinander beabstandeten Teilbereichen besteht. Diese beiden Teilbereiche der Gleitfläche 96 am distalen Ende der Antriebseinrichtung 90 berühren die Gleitflächen 64 am Gehäuse der Bilderfassungseinrichtung 50 an zwei voneinander beabstandeten Orten. An jeder der beiden Teilbereiche der Gleitfläche 96 ist die zugeordnete Flächennormale 97 orthogonal zur Längsachse des Schafts 12 und damit parallel zur Schnittebene der Figur 7.

Auch bei dem anhand der Figuren 1 bis 4 dargestellten Endoskop kann die Gleitfläche 96 am distalen Ende 92 der Antriebseinrichtung 90 aus zwei oder mehr voneinander beabstandeten Teilbereichen bestehen.

Fig. 8 zeigt eine schematische Darstellung eines distalen Endes 14 eines Schafts 12 eines weiteren Endoskops 10, das in einigen Merkmalen, Eigenschaften und Funktionen den anhand der Figuren 1 bis 7 dargestellten Endoskopen ähnelt. Nachfolgend sind insbesondere Merkmale, Eigenschaften und Funktionen beschrieben, in denen das in Figur 8 gezeigte Endoskop 10 sich von den anhand der Figuren 1 bis 7 dargestellten Endoskopen unterscheidet. Die Art der Darstellung in Figur 8, insbesondere die Orientierung der Zeichenebene und die Lage und Orientierung der Schnittebene des Schafts 12, entspricht derjenigen der Figuren 2 bis 5.

Das in Figur 8 gezeigte Endoskop unterscheidet sich von den anhand der Figuren 1 bis 7 dargestellten Endoskopen insbesondere dadurch, dass die Gleitfläche 64 an dem Gehäuse 60 der Bilderfassungseinrichtung 50 im Wesentlichen nach proximal und die Gleitfläche 96 am distalen Ende 92 der Antriebseinrichtung 90 im Wesentlichen nach distal orientiert sind. Die Flächennormale 97 der Gleitfläche 96 am distalen Ende der Antriebseinrichtung 90 ist bei dem dargestellten Beispiel parallel zu der Längsachse 38 des Schafts 12. Die Gleitfläche 96 an dem distalen Ende 92 der Antriebseinrichtung 90 kann zwei oder mehr voneinander beabstandete Teilbereiche aufweisen, ähnlich wie bei dem anhand der Figur 7 dargestellten Beispiel.

Bei allen anhand der Figuren 1 bis 8 dargestellten Endoskopen kann die Bilderfassungseinrichtung 50 zwei oder mehr nebeneinander angeordnete Objektive 52 und optional eine entsprechende Anzahl von Bildsensoren aufweisen, um ein Stereobild erfassen zu können. In diesem Fall kann das Kugelgelenk 62 in einem Bereich zwischen den Objektiven 52 oder zwischen den Bildsensoren 54 angeordnet sein.

Bei allen anhand der Figuren 1 bis 8 dargestellten Endoskopen kann anstelle des Kugelgelenks 62 eine andere Schwenklagereinrichtung vorgesehen sein, beispielsweise in Gestalt einer kardanischen Aufhängung.

### Bezugszeichen

- **10**: **Endoskop**
- 12: Schaft des Endoskops 10
- 14: distales Ende des Schafts 12
- 16: proximales Ende des Schafts 12
- 18: proximaler Bereich des Endoskops 10
- 19: Drehrad als Bedienelement des Endoskops 10
- 38: Längsachse des Schafts 12 und Rotationsachse der Antriebseinrichtung 90
- **40**: **Fensterbauteil** an dem distalen Ende 24 des Schafts 20
- 48: Flächennormale des Fensterbauteils 40
- **50**: **Bilderfassungseinrichtung** des Endoskops 10
- 52: Objektiv der Bilderfassungeinrichtung 50, zum Erzeugen eines reellen Bilds
- 54: Bildsensor der Bilderfassungseinrichtung 50, zum Erfassen des von dem Objektiv 52 erzeugten reellen Bilds und zum Erzeugen eines Bildsignals
- 56: Signalleitung zum Übertragen von Leistung und/oder eines Steuersignals zu dem Bildsensor 54 und/oder zum Übertragen des Bildsignals von dem Bildsensor 54
- 58: optische Achse des Objektivs 52 und Blickrichtung der Bilderfassungseinrichtung 50
- **60**: **Gehäuse** der Bilderfassungseinrichtungen 50
- 62: Schwenklagereinrichtung, insbesondere Kugelgelenk zwischen der Bilderfassungseinrichtung 50 und dem distalen Ende 14 des Schafts 12
- 64: Gleitfläche an dem Gehäuse 60
- 86: stabförmiges Bauteil, das das Schwenklager 62 mit dem Schaft 12 mechanisch starr verbindet
- 88: Schlitz in dem Gehäuse 60, in dem das stabförmige Bauteil 86 angeordnet ist
- **90**: **Antriebseinrichtung** des Endoskops 10
- 92: distales Ende der Antriebseinrichtung 90
- 96: Gleitfläche an dem distalen Ende 92 der Antriebseinrichtung 90
- 97: Flächennormale der Gleitfläche 96
- 98: Lumen der Antriebseinrichtung 90

## Patentansprüche

1. Endoskop (10) mit:
einer Bilderfassungseinrichtung (50) mit einem Objektiv (52) zum Erzeugen eines reellen Bilds und einem Bildsensor (54) zum Erfassen des reellen Bilds und zum Erzeugen eines Bildsignals, das das erfasste reelle Bild repräsentiert;
einer Schwenklagereinrichtung (62), die ein Bewegen der optischen Achse (58) des Objektivs (52) der Bilderfassungseinrichtung (50) auf einem Kegelmantel ermöglicht;
einer um eine Rotationsachse (38) rotierbaren Antriebseinrichtung (90),
wobei die rotierbare Antriebseinrichtung (90) derart mit der Bilderfassungseinrichtung (50) mechanisch gekoppelt ist, dass eine Rotation der rotierbaren Antriebseinrichtung (90) mit einem Bewegen der optischen Achse (58) des Objektivs (54) der Bilderfassungseinrichtung (50) auf einem Kegelmantel einher geht,
wobei, bei dem die Schwenklagereinrichtung (62) ein Schwenken um eine erste Schwenkachse orthogonal oder im Wesentlichen othogonal zu einer Symmetrieachse des Kegelmantels und ein Schwenken um eine zweite Schwenkachse orthogonal oder im Wesentlichen orthogonal zu der ersten Schwenkachse und orthogonal oder im Wesentlichen orthogonal zu der Symmetrieachse des Kegelmantels ermöglicht,
ferner mit einer ersten Gleitfläche (64), die mit der Bilderfassungseinrichtung (50) mechanisch starr verbunden ist; und
einer zweiten Gleitfläche (96) an einem distalen Ende (92) der rotierbaren Antriebseinrichtung (90), **dadurch gekennzeichnet, dass** die zweite Gleitfläche (96) an der ersten Gleitfläche (64) anliegt.

2. Endoskop (10) nach dem vorangehenden Anspruch, bei dem ein distales Ende (92) der Antriebseinrichtung (90) zumindest entweder proximal der Schwenklagereinrichtung (62) oder proximal der Bilderfassungseinrichtung (50) angeordnet ist.

3. Endoskop (10) nach einem der vorangehenden Ansprüche, ferner mit:
einer Führungseinrichtung (86, 88), die eine vorbestimmte Reihe von Pixeln des Bildsensors (54) in jeder Position der Bilderfassungseinrichtung (50) parallel zu einer vorbestimmten Ebene hält.

4. Endoskop (10) nach einem der vorangehenden Ansprüche, bei dem die Schwenklagereinrichtung ein Kugelgelenk (62), ein stabförmiges Bauteil (86), an dessen Ende das Kugelgelenk (62) angeordnet ist, und einen Schlitz (88), in dem das stabförmige Bauteil (86) spiel- und reibungsarm schwenkbar geführt ist, umfasst.

5. Endoskop (10) nach dem vorangehenden Anspruch, bei dem die erste Gleitfläche (64) kreisbogenförmig oder kreisringförmig ist.

6. Endoskop (10) nach einem der Ansprüche 1 und 5 , bei dem die erste Gleitfläche (64) rotationssymmetrisch zur Blickrichtung (58) der Bilderfassungseinrichtung (50) ist.

7. Endoskop (10) nach einem der Ansprüche 1 und 5 bis 6, bei dem die zweite Gleitfläche (96) zwei von einander beabstandete Teilflächen umfasst.

8. Endoskop (10) nach einem Ansprüche 1 und 5 bis 7, bei dem die erste Gleitfläche (64) nach proximal und die zweite Gleitfläche (96) nach distal orientiert sind.

9. Endoskop (10) nach einem Ansprüche 1 und 5 bis 8 , bei dem eine Flächennormale der zweiten Gleitfläche (96) zu einer Rotationsachse (38) der rotierbaren Antriebseinrichtung (90) hin orientiert ist.

10. Endoskop (10) nach einem der vorangehenden Ansprüche, wobei zwei gleiche oder ähnliche Bilderfassungseinrichtungen (50) nebeneinander angeordnet und miteinander mechanisch starr verbunden sind.

11. Endoskop (10) nach dem vorangehenden Anspruch, bei dem die Schwenklagereinrichtung ein Kugelgelenk (62) umfasst, das zwischen den Bilderfassungseinrichtungen (50) angeordnet ist.

12. Endoskop (10) nach einem der vorangehenden Ansprüche, ferner mit:
einem Schaft (12) mit einem proximalen Ende (16) und einem distalen Ende (14) zum Einführen in einen Hohlraum,
einer Signalleitung (56) zumindest entweder zum Übertragen eines Steuersignals von dem proximalen Ende (16) des Schafts (12) zu der Bilderfassungseinrichtung (50) oder zum Übertragen eines Bildsignals von der Bilderfassungseinrichtung (50) zu dem proximalen Ende (16) des Schafts (12),
wobei die rotierbare Antriebseinrichtung (90) als Rohr ausgebildet ist,
wobei die Signalleitung (56) innerhalb eines Lumens der rotierbaren Antriebseinrichtung (90) angeordnet ist.

## Claims

1. An endoscope (10) having:
an image capturing apparatus (50) having an objective (52) for generating a real image and an image sensor (54) for capturing the real image and generating an image signal representing the captured real image;
a pivot bearing apparatus (62) which enables the optical axis (58) of the objective (52) of the image capturing apparatus (50) to be moved on a cone surface;
a drive apparatus (90) rotatable about an axis of rotation (38),
wherein the rotatable drive apparatus (90) is mechanically coupled to the image capturing apparatus (50) in such manner that a rotation of the rotatable drive apparatus (90) is accompanied by the optical axis (58) of the objective (54) of the image capturing apparatus (50) moving on a cone surface,
wherein the pivot bearing apparatus (62) enables pivoting about a first pivot axis orthogonal or substantially orthogonal to an axis of symmetry of the cone surface and pivoting about a second pivot axis orthogonal or substantially orthogonal to the first pivot axis and orthogonal or substantially orthogonal to the axis of symmetry of the cone surface,
further having a first sliding surface (64) which is mechanically rigidly connected to the image capturing apparatus (50); and
a second sliding surface (96) at a distal end (92) of the rotatable drive apparatus (90), **characterised in that** the second sliding surface (96) bears against the first sliding surface (64).

2. The endoscope (10) according to the preceding claim, in which a distal end (92) of the drive apparatus (90) is arranged at least either proximally of the pivot bearing apparatus (62) or proximally of the image capturing apparatus (50).

3. The endoscope (10) according to one of the preceding claims, further having:
a guide apparatus (86, 88) which holds a predetermined row of pixels of the image sensor (54) parallel to a predetermined plane at each position of the image capturing apparatus (50).

4. The endoscope (10) according to one of the preceding claims, in which the pivot bearing apparatus comprises a ball joint (62), a rod-shaped component (86), at the end of which the ball joint (62) is arranged, and a slit (88), in which the rod-shaped component (86) is guided so as to pivot with little play and friction.

5. The endoscope (10) according to the preceding claim, in which the first sliding surface (64) is arc-shaped or ring-shaped.

6. The endoscope (10) according to one of claims 1 and 5, in which the first sliding surface (64) is rotationally symmetrical to the viewing direction (58) of the image capturing apparatus (50).

7. The endoscope (10) according to one of claims 1 and 5 to 6, in which the second sliding surface (96) comprises two partial surfaces spaced apart from each other.

8. The endoscope (10) according to one of claims 1 and 5 to 7, in which the first sliding surface (64) is orientated proximally and the second sliding surface (96) is orientated distally.

9. The endoscope (10) according to one of claims 1 and 5 to 8, in which a surface normal of the second sliding surface (96) is orientated towards an axis of rotation (38) of the rotatable drive apparatus (90).

10. The endoscope (10) according to one of the preceding claims, wherein two identical or similar image capturing apparatuses (50) are arranged next to each other and are mechanically rigidly connected to each other.

11. The endoscope (10) according to the preceding claim, in which the pivot bearing apparatus comprises a ball joint (62) which is arranged between the image capturing apparatuses (50).

12. The endoscope (10) according to one of the preceding claims, further having:
a shaft (12) with a proximal end (16) and a distal end (14) for insertion into a cavity,
a signal line (56) at least either for transmitting a control signal from the proximal end (16) of the shaft (12) to the image capturing apparatus (50) or for transmitting an image signal from the image capturing apparatus (50) to the proximal end (16) of the shaft (12),
wherein the rotatable drive apparatus (90) is designed as a tube, wherein the signal line (56) is arranged inside a lumen of the rotatable drive apparatus (90).

## Revendications

1. Endoscope (10) comportant :
un dispositif d'acquisition d'image (50) comportant un objectif (52) pour la génération d'une image réelle et un capteur d'image (54) pour l'acquisition de l'image réelle et pour la génération d'un signal d'image, qui représente l'image réelle acquise ;
un dispositif de palier pivotant (62) qui permet un mouvement de l'axe optique (58) de l'objectif (52) du dispositif d'acquisition d'image (50) sur une enveloppe conique ;
un dispositif d'entraînement (90) rotatif autour d'un axe de rotation (38),
dans lequel le dispositif d'entraînement (90) rotatif est accouplé mécaniquement au dispositif d'acquisition d'image (50) de telle sorte qu'une rotation du dispositif d'entraînement (90) rotatif s'accompagne d'un mouvement de l'axe optique (58) de l'objectif (54) du dispositif d'acquisition d'image (50) sur une enveloppe conique,
dans lequel le dispositif de palier pivotant (62) permet un pivotement autour d'un premier axe de pivotement orthogonal ou sensiblement orthogonal à un axe de symétrie de l'enveloppe conique et un pivotement autour d'un second axe de pivotement orthogonal ou sensiblement orthogonal au premier axe de pivotement et orthogonal ou sensiblement orthogonal à l'axe de symétrie de l'enveloppe conique,
comportant en outre une première surface de glissement (64) reliée mécaniquement de manière rigide au dispositif d'acquisition d'image (50) ; et
une seconde surface de glissement (96) à une extrémité distale (92) du dispositif d'entraînement (90) rotatif, **caractérisé en ce que** la seconde surface de glissement (96) s'appuie sur la première surface de glissement (64).

2. Endoscope (10) selon la revendication précédente, dans lequel une extrémité distale (92) du dispositif d'entraînement (90) est agencée au moins soit de manière proximale par rapport au dispositif de palier pivotant (62), soit de manière proximale par rapport au dispositif d'acquisition d'image (50).

3. Endoscope (10) selon l'une des revendications précédentes, comportant en outre :
un dispositif de guidage (86, 88) qui maintient une rangée prédéterminée de pixels du capteur d'image (54) parallèle à un plan prédéterminé dans chaque position du dispositif d'acquisition d'image (50).

4. Endoscope (10) selon l'une des revendications précédentes, dans lequel le dispositif de palier pivotant comprend une articulation à rotule (62), un composant en forme de tige (86) à l'extrémité duquel est agencée l'articulation à rotule (62), et une fente (88) dans laquelle le composant en forme de tige (86) est guidé de manière à pouvoir pivoter sans jeu et sans frottement.

5. Endoscope (10) selon la revendication précédente, dans lequel la première surface de glissement (64) est en forme d'arc de cercle ou d'anneau de cercle.

6. Endoscope (10) selon l'une des revendications 1 et 5, dans lequel la première surface de glissement (64) présente une symétrie de rotation par rapport à la direction de visée (58) du dispositif d'acquisition d'images (50).

7. Endoscope (10) selon l'une des revendications 1 et 5 à 6, dans lequel la seconde surface de glissement (96) comprend deux surfaces partielles espacées l'une de l'autre.

8. Endoscope (10) selon l'une des revendications 1 et 5 à 7, dans lequel la première surface de glissement (64) est orientée vers le côté proximal et la seconde surface de glissement (96) est orientée vers le côté distal.

9. Endoscope (10) selon l'une des revendications 1 et 5 à 8, dans lequel une normale à la surface de la seconde surface de glissement (96) est orientée vers un axe de rotation (38) du dispositif d'entraînement (90) rotatif.

10. Endoscope (10) selon l'une des revendications précédentes, dans lequel deux dispositifs d'acquisition d'images (50) identiques ou similaires sont agencés côte à côte et sont reliés mécaniquement entre eux de manière rigide.

11. Endoscope (10) selon la revendication précédente, dans lequel le dispositif de palier pivotant comprend une articulation à rotule (62) qui est agencée entre les dispositifs d'acquisition d'image (50).

12. Endoscope (10) selon l'une des revendications précédentes, comportant en outre :
une tige (12) comportant une extrémité proximale (16) et une extrémité distale (14) destinée à être insérée dans une cavité,
une ligne de signal (56) au moins soit pour transmettre un signal de commande de l'extrémité proximale (16) de la tige (12) au dispositif d'acquisition d'image (50), soit pour transmettre un signal d'image du dispositif d'acquisition d'image (50) à l'extrémité proximale (16) de la tige (12),
dans lequel le dispositif d'entraînement (90) rotatif est conçu comme un tube, dans lequel la ligne de signal (56) est agencée à l'intérieur d'une lumière du dispositif d'entraînement (90) rotatif.
